# FASCICULE DE BREVET EUROPEEN

(11) **EP 2 547 395 B1**
(45) Date de publication et mention de la délivrance du brevet: **04.01.2017**
(21) Numéro de dépôt: 11715575.4
(22) Date de dépôt: 15.03.2011
(51) Int. Cl.: A61N 1/05, A61B 5/04, H01L 21/48, A61B 5/0478

(54) **PROCÉDÉ DE FABRICATION D'UNE ELECTRODE A USAGE MÉDICAL ET ÉLECTRODE OBTENUE PAR LA MISE EN OEUVRE DE CE PROCÉDÉ**
VERFAHREN ZUR HERSTELLUNG EINER ELEKTRODE FÜR MEDIZINISCHE ZWECKE UND DURCH ANWENDUNG DIESES VERFAHRENS GEWONNENE ELEKTRODE
PROCESS FOR MANUFACTURING AN ELECTRODE FOR MEDICAL USE AND ELECTRODE OBTAINED BY THE IMPLEMENTATION OF THIS PROCESS

(30) Priorité: 18.03.2010 FR 1051942
(43) Date de publication de la demande: 23.01.2013
(73) Titulaire: Dixi Microtechniques, 25025 Besançon Cedex (FR)
(72) Inventeur: BOILLON, Christophe, 25640 Pouligney (FR)
(74) Mandataire: Koelbel, Caroline
(86) Numéro de dépôt international: PCT/FR2011/000141
(87) Numéro de publication internationale: WO 2011/114020

(56) Documents cités:
- WO-A1-2005/004978
- US-A- 5 720 099
- US-A1- 2007 005 112
- US-A1- 2007 007 240

## Description

La présente invention concerne un procédé de fabrication d'une électrode à usage médical, telle qu'une électrode corticale destinée à être utilisée au niveau du cerveau, dans lequel pour réaliser ladite électrode, on utilise une bande de silicone pour former un substrat souple, on place sur ledit substrat souple un premier masque déterminant un motif agencé pour définir au moins une piste électrique présentant au moins un plot de contact.

La présente invention concerne également une électrode à usage médical, telle qu'une électrode corticale destinée à être utilisée au niveau du cerveau, ladite électrode comportant une bande de silicone formant un substrat souple sur lequel est déposée au moins une couche de métal agencée pour définir au moins une piste électrique présentant au moins un plot de contact.

### Technique antérieure :

Les électrodes corticales sont des dispositifs utilisés, selon les cas, à des fins de diagnostic, dans un but thérapeutique, ou pour réaliser des études. Ainsi, elles sont couramment utilisées pour réaliser des enregistrements par électroencéphalogramme, par exemple dans le but de localiser des dysfonctionnements du cerveau et établir un diagnostic pré-chirurgical d'épilepsies résistantes aux traitements médicamenteux, ou dans le but d'obtenir une cartographie neurophysiologique, lors d'interventions neurochirurgicales. Elles sont également largement employées pour réaliser des stimulations directes intracérébrales, se révélant bénéfiques dans certaines pathologies comme les syndromes douloureux, ou destinées à déclencher des auras, ou autres crises, à des fins d'observation.

En fonction du cas de figure, ces électrodes sont simplement disposées au niveau du scalp, ou placées directement au contact du cerveau, au travers d'orifices réalisés dans la boîte crânienne.

Un exemple d'électrodes corticales disponible sur le marché à l'heure actuelle se présente sous la forme de pastilles métalliques en platine/iridium reliées, au travers de fils électriques eux-mêmes en platine/iridium, aux appareils électriques d'enregistrements ou de stimulations appropriés. Ces pastilles, connectées chacune à un fil électrique, sont disposées sur un support fin et souple, d'une manière telle qu'elles défnissent une grille apte à épouser parfaitement la forme des zones à explorer. Une telle grille est de configuration variable, et peut notamment être prédécoupée pour offrir le nombre de contacts électriques adapté à la surface de la zone du cerveau concernée.

Un procédé de fabrication d'une telle grille d'électrodes corticales consiste, dans un premier temps, à disposer les pastilles métalliques sur un gabarit, à les connecter individuellement, par soudure, sur les fils électriques préalablement introduits dans une gaine silicone et, dans un second temps, à placer l'ensemble pastilles-fils électriques en sandwich entre deux feuilles de silicone ultérieurement collées entre elles puis découpées pour obtenir la forme finale de la grille.

Un tel procédé ne donne pas entière satisfaction et se révèle fastidieux et coûteux en raison du nombre élevé d'étapes qu'il implique, notamment pour connecter chaque pastille à un fil électrique. Par ailleurs, les matières premières employées pour fabriquer les pastilles et les fils électriques, à savoir un alliage platine/iridium, sont également connues pour leur prix élevé, se répercutant inéluctablement, au final, sur celui des grilles d'électrodes corticales elles-mêmes.

Le document US 6,624,510 décrit par ailleurs un autre exemple d'électrode corticale comprenant un substrat flexible, de préférence une couche de polyimide, sur laquelle sont déposées par évaporation sous vide ou par dépôt électrolytique, des couches d'un ou de plusieurs métaux. Chaque électrode comprend un plot de contact en platine relié par une zone de raccordement à un appareil d'enregistrement au travers d'une piste électrique fabriquée à partir d'une couche de titane recouverte d'une couche d'or. L'ensemble, à l'exception des plots de contact et de la zone de raccordement, est recouvert d'un film électriquement isolant, tel qu'un film de silicone. Le procédé de fabrication décrit dans ce document ne donne pas entière satisfaction, du fait qu'il nécessite un nombre important d'étapes délicates à réaliser et qu'il repose sur l'utilisation de matériaux onéreux.

La publication US 2007/0007240, ayant pour objet un procédé de fabrication d'une microélectrode intra-corticale pourvue d'un connecteur souple, repose quant à elle sur la technique de fabrication des semi-conducteurs CMOS. Une variante de réalisation de ce procédé prévoit de réaliser un connecteur sur un substrat semi-conducteur, en déposant une couche conductrice pouvant comprendre de l'or sur un substrat en silicium, de recouvrir le connecteur avec une couche de polymère déposée en phase vapeur à travers un masque, puis d'immerger le substrat semi-conducteur et le connecteur recouvert de la couche de polymère dans un bain de décapage, et de retirer le substrat semi-conducteur du connecteur ainsi fabriqué pour qu'il devienne souple. Un tel procédé présente l'inconvénient d'être complexe et onéreux puisqu'il nécessite de nombreuses étapes et de nombreux masques. Il utilise également des procédés de décapage en phase liquide ou solide.

Un autre procédé, décrit dans la publication US 2007/0005112 prévoit de réaliser une connexion entre un boîtier électronique et une électrode corticale en déposant un métal biocompatible, tel que du platine ou de l'or, par galvanoplastie, sur un substrat en polyimide qui ne présente aucune élasticité lui permettant de s'adapter aux formes arrondies d'un crâne.

Par ailleurs, on connaît de la publication US 2007/0007240 A1 un procédé de fabrication d'électrodes microstructurées qui peuvent être utilisées dans le cerveau. Elles sont obtenues en déposant sur un substrat semi-conducteur des couches de silicone, des couches d'un matériau conducteur, tel que du métal et des couches de polymère, tel que du parylène. La couche de polymère est déposée par dépôt physique en phase vapeur puis gravée. Il en résulte une électrode intracérébrale flexible formée par deux connecteurs reliés entre eux par un câble en polymère et enrobé de ce polymère souple. Un tel procédé est onéreux et nécessite l'utilisation d'un substrat en semi-conducteur devant être ultérieurement retiré.

### Exposé de l'invention :

La présente invention vise à pallier ces inconvénients en proposant un procédé simplifié de fabrication d'une électrode corticale, impliquant un nombre limité de manipulations et d'étapes et basé sur l'utilisation de matériaux moins coûteux, ce procédé pouvant être facilement industrialisé.

Dans ce but, l'invention concerne un procédé du genre indiqué en préambule, dans lequel l'on rigidifie ladite bande de silicone en déposant sur au moins une de ses faces une couche d'un polymère et en ce que ledit premier masque est fabriqué à partir d'une feuille d'un métal ou d'un alliage de métaux, on dispose une pièce aimantée sur la face du substrat souple opposée à celle sur laquelle ledit masque est appliqué, de manière à obtenir une étanchéité entre ledit substrat souple et ledit premier masque et on dépose une couche de métal sur ledit substrat souple à travers ledit masque par une technique de dépôt physique en phase vapeur.

Selon une caractéristique de ce procédé, l'on utilise une bande de silicone du type présentant une structure renforcée

Selon une autre caractéristique du présent procédé, l'on utilise en tant que masque une feuille d'un métal ou d'un alliage de métaux choisi(s) dans le groupe comprenant du molybdène, de l'acier inoxydable, du nickel, présentant une épaisseur de préférence comprise entre 50µm et 200µm.

Par ailleurs, le présent procédé se caractérise encore en ce que l'on active chimiquement la zone du substrat souple non recouverte par ledit masque.

Dans ce cas, pour activer ladite bande de silicone on le soumet à une étape de nettoyage ionique effectué au moyen d'un mélange d'oxygène et d'argon à l'état de plasma, puis on y dépose une couche de titane.

Selon une autre caractéristique du procédé selon l'invention, l'on utilise en tant que métal pour définir ladite piste électrique, un métal noble, ou un alliage de métaux nobles.

Une caractéristique additionnelle du présent procédé prévoit encore que l'on recouvre l'ensemble formé par ledit substrat souple et ladite piste électrique, à l'exception du plot de contact, d'une couche d'un matériau protecteur, déposée à travers un second masque par une technique de dépôt chimique en phase vapeur.

Dans ce cas, ledit polymère ou ledit matériau formant ladite couche est du parylène.

L'invention concerne également une électrode à usage médical du genre indiqué en préambule, dans laquelle ladite bande de silicone est recouverte sur au moins une de ses faces d'une couche d'un polymère rigidifiant

Selon une forme de réalisation préférentielle, la bande de silicone utilisée présente une structure renforcée, et de préférence, une épaisseur d'au moins 200 µm.

Dans ce cas, ledit polymère est du parylène, dont l'épaisseur présente une valeur comprise entre 0,5µm et 10µm.

D'autre part, l'électrode selon l'invention se caractérise également en ce que le métal, définissant au moins une piste électrique présentant au moins un plot de contact, est un métal noble, ou un alliage de métaux nobles.

De préférence, la couche de métal présente une épaisseur d'au moins 400 nm.

Une caractéristique additionnelle de la présente invention est encore définie par le fait que ladite électrode est recouverte, à l'exception des plots de contact, par une couche d'un matériau protecteur, par exemple du parylène présentant de préférence une épaisseur d'au moins 1 µm.

### Description sommaire des dessins :

La présente invention et ses avantages apparaîtront mieux dans la description suivante d'un mode de réalisation donné à titre d'exemple non limitatif, en référence aux dessins annexés, dans lesquels:
- la figure 1 représente une vue en coupe d'une électrode selon l'invention en cours de fabrication, selon le plan de coupe AA de la figure 3,
- la figure 2 est une vue en coupe de l'électrode représentée sur la figure 1, finalisée, selon le plan de coupe AA de la figure 3, et
- la figure 3 est une vue de dessus d'un exemple de réalisation d'une électrode selon l'invention.

### Illustrations de invention et meilleure manière de la réaliser:

En référence aux figures, la présente invention a pour objet un procédé de fabrication d'une électrode corticale 1, consistant à déposer une couche d'un métal sur un substrat souple constitué par une bande de silicone 3 de sorte à définir au moins une piste conductrice 2, présentant au moins un plot de contact 20.

La silicone est un matériau souple, flexible et biocompatible, avantageusement capable d'épouser une forme sphérique et présentant une partie hydrophile permettant une bonne adhésion sur la surface du cortex cérébral.

De préférence, la silicone choisie pour la mise en oeuvre du présent procédé est du type présentant une structure renforcée, par exemple par l'insertion d'une maille textile en polyester lors de son extrusion. Il est en outre prévu d'utiliser une bande de silicone 3 ayant de préférence une épaisseur d'au moins 200µm, par exemple 300µm, rigidifiée par l'application d'une couche 4 d'un polymère biocompatible, tel que par exemple du parylène, agencée pour supprimer au moins en partie l'élasticité de la silicone. Bien entendu, tout autre polymère biocompatible, apte à rigidifier et lisser la surface de la silicone, pourrait être employé dans le même but de supprimer au moins en partie l'élasticité de la silicone pour éviter tout risque de microcoupure ou cassure des pistes conductrices déposées.

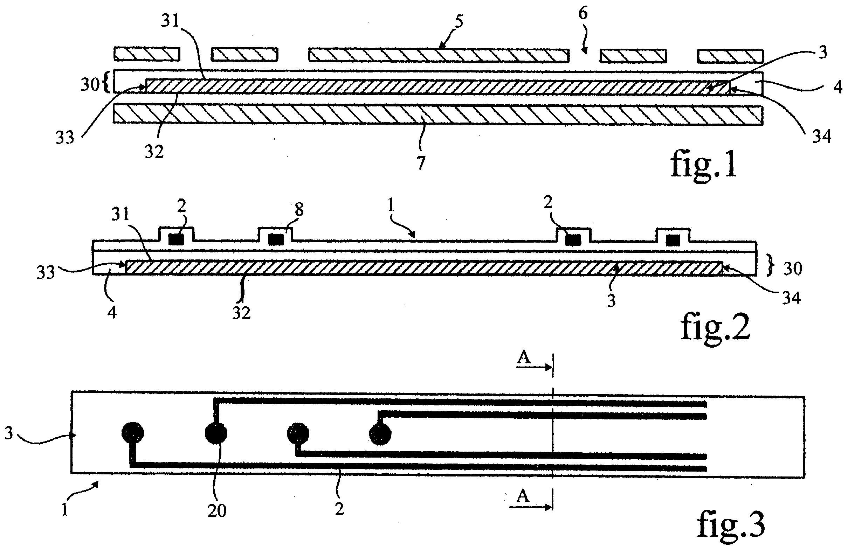

Conformément au présent procédé, la couche 4 de parylène est appliquée, par exemple par dépôt chimique en phase vapeur, sur une épaisseur comprise par exemple entre 0,5µm et 10µm, de préférence 1µm, de manière à recouvrir au moins les bords 33, 34 de la bande de silicone 3 (cf. fig.1 et 2) ainsi que la face 31 supposée porter ladite piste conductrice 2.

Un masque 5, comportant un motif 6 agencé pour définir, dans l'exemple représenté, une pluralité de pistes électriques 2 présentant chacune au moins un plot de contact 20, est ensuite placé en regard de la face 31 du substrat souple 30 défini par la bande de silicone 3 et la couche de parylène 4.

Selon l'invention, ce masque 5 est fabriqué à partir d'une feuille d'un métal ou d'un alliage de métaux, choisi(s) dans le groupe comprenant du molybdène, de l'acier inoxydable, du nickel, ou similaire, cette feuille présentant une épaisseur comprise par exemple entre 50µm et 200µm. Le motif 6 est réalisé dans le masque 5 en découpant ladite feuille par gravure au laser, ou en utilisant toute autre technique similaire.

De préférence, et dans le but d'obtenir une parfaite étanchéité entre le substrat 30 et le masque 5, on utilise un masque 5 réalisé à partir d'une feuille comprenant du nickel, et l'on dispose une plaque aimantée 7 contre l'autre face 32 dudit substrat 30 pour plaquer le masque 5 sur le substrat 30.

L'ensemble ainsi obtenu est ensuite placé dans une enceinte agencée pour effectuer un dépôt physique en phase vapeur d'une couche d'au moins un métal sur ledit substrat 30, à travers le motif 6 que comporte le masque 5.

Préalablement à l'étape consistant à réaliser ledit dépôt de métal, le présent procédé préconise encore d'activer chimiquement la zone du substrat 30 non recouverte par ledit masque 5, c'est à dire la zone correspondant au motif 6. Ce but est atteint en effectuant un nettoyage ionique, au moyen d'un mélange d'oxygène et d'argon, puis en déposant une couche de titane de préférence sur une épaisseur d'au moins 400nm sur ladite zone. La couche de titane a l'avantage d'améliorer l'adhérence de la couche de métal sur le substrat 30.

Enfin, une couche de métal noble, par exemple de l'or, de préférence d'une épaisseur d'au moins 400nm est déposée sur le substrat 30, à travers le motif 6 du masque 5, par une technique de dépôt physique en phase vapeur, telle que, par exemple, la technique de pulvérisation cathodique magnétron.

L'or présente l'avantage d'être inoxydable et est donc adapté pour un contact direct avec la surface du cerveau. Il se caractérise par ailleurs par une bonne conductivité, et autorise en outre la visualisation des pistes conductrices, notamment par Imagerie par Résonance Magnétique (IRM) ou par rayons-X. Néanmoins, il peut bien entendu être remplacé par un autre métal noble présentant des propriétés équivalentes, tel que le platine, l'iridium, le rhodium et l'argent. En outre un alliage de métaux nobles, tels que par exemple le platine iridium ou l'électrum pourrait également convenir.

Après retrait de l'aimant 7 et du masque 5, le présent procédé prévoit encore de recouvrir l'ensemble formé par ledit substrat souple 30 et les pistes électriques 2, à l'exception des plots de contact 20, d'une couche 8 d'un matériau protecteur, tel que notamment du parylène, ou tout autre matériau présentant des propriétés protectrices et isolantes similaires.

Ce dépôt d'une couche 8 de parylène est réalisé par exemple par une technique de dépôt chimique en phase vapeur, à travers un second masque, non représenté, agencé pour recouvrir les plots 20 et permettre l'exposition desdites pistes 2. Par ailleurs, ladite couche 8 présente de préférence une épaisseur d'au moins 1 µm.

Avantageusement, le procédé selon invention prévoit encore d'effectuer une stérilisation de l'électrode corticale 1 ainsi obtenue, par exemple à l'oxyde d'éthylène.

### Possibilités d'application industrielle :

Il ressort clairement de cette description que le nombre réduit d'étapes que comporte le présent procédé ainsi que les matériaux employés permettent d'atteindre les buts fixés, à savoir faciliter la fabrication d'une électrode corticale et en réduire les coûts.

Compte tenu de son coût avantageux, l'électrode corticale 1 ainsi obtenue peut être à usage unique, rendant son utilisation entièrement sécurisée et supprimant le recours aux techniques de stérilisation lourdes et coûteuses.

Par ailleurs, le présent procédé repose sur la mise en oeuvre de techniques adaptées à une industrialisation de la fabrication, et les matériaux employés peuvent être recyclés, ce qui est particulièrement avantageux, notamment en ce qui concerne les métaux.

La présente invention n'est pas limitée à l'exemple de réalisation décrit mais s'étend à toute modification et variante évidentes pour un homme du métier tout en restant dans l'étendue de la protection définie dans les revendications annexées.

## Revendications

1. Procédé de fabrication d'une électrode à usage médical, telle qu'une électrode corticale (1) destinée à être utilisée au niveau du cerveau, dans lequel pour réaliser ladite électrode, on utilise une bande de silicone (3) pour former un substrat souple (30), on place sur ledit substrat souple (30) un premier masque (5) déterminant un motif (6) agencé pour définir au moins une piste électrique (2) présentant au moins un plot de contact (20), **caractérisé en ce que** l'on rigidifie ladite bande de silicone (3) en déposant sur au moins une de ses faces une couche (4) d'un polymère, et **en ce que** ledit premier masque (5) est fabriqué à partir d'une feuille d'un métal ou d'un alliage de métaux et on dispose une pièce aimantée (7) sur la face (32) du substrat souple (30) opposée à celle sur laquelle ledit masque (5) est appliqué, de manière à obtenir une étanchéité entre ledit substrat souple (30) et ledit premier masque (5), et on dépose une couche de métal sur ledit substrat souple (30) à travers ledit masque (5) par une technique de dépôt physique en phase vapeur.

2. Procédé selon la revendication 1, **caractérisé en ce que** l'on utilise une bande de silicone (3) du type présentant une structure renforcée.

3. Procédé selon la revendication 1, **caractérisé en ce que** l'on utilise en tant que premier masque (5) une feuille d'un métal ou d'un alliage de métaux choisi(s) dans le groupe comprenant du molybdène, de l'acier inoxydable, du nickel.

4. Procédé selon la revendication 3, **caractérisé en ce que** l'on utilise une feuille d'un métal ou d'un alliage de métaux présentant une épaisseur comprise entre 50µm et 200µm.

5. Procédé selon la revendication 1, **caractérisé en ce que**, préalablement à l'étape consistant à réaliser ledit dépôt de métal, l'on active chimiquement la zone du substrat souple (30) non recouverte par ledit masque (5).

6. Procédé selon la revendication 5, **caractérisé en ce que**, pour activer ledit substrat souple (30), on le soumet à une étape de nettoyage ionique effectué au moyen d'un mélange d'oxygène et d'argon à l'état de plasma, puis on y dépose une couche de titane.

7. Procédé selon l'une quelconque des revendications précédentes, **caractérisé en ce que** l'on utilise, en tant que métal pour définir ladite piste électrique (2), un métal noble, ou un alliage de métaux nobles.

8. Procédé selon l'une quelconque des revendications précédentes, **caractérisé en ce que** l'on recouvre l'ensemble formé par ledit substrat souple (30) et ladite piste électrique (2), à l'exception du plot de contact (20), d'une couche (8) d'un matériau protecteur, déposée à travers un second masque par une technique de dépôt chimique en phase vapeur.

9. Procédé selon l'une quelconque des revendications 1 ou 8, **caractérisé en ce que** ledit polymère formant ladite couche (4) ou ledit matériau protecteur formant ladite couche (8) est du parylène.

10. Electrode (1) à usage médical, obtenue par la mise en oeuvre du procédé selon l'une quelconque des revendications 1 à 9, telle qu'une électrode corticale (1) destinée à être utilisée au niveau du cerveau, ladite électrode comportant une bande (3) de silicone formant un substrat souple (30) sur lequel est déposée au moins une couche de métal agencée pour définir au moins une piste électrique (2) présentant au moins un plot de contact (20), caractérisée en ce ladite bande (3) de silicone est recouverte sur au moins une de ses faces d'une couche (4) d'un polymère rigidifiant.

11. Electrode selon la revendication 10, **caractérisée en ce que** la bande (3) de silicone utilisée présente une structure renforcée.

12. Electrode selon la revendication 11, **caractérisée en ce que** ladite bande (3) de silicone présente une épaisseur d'au moins 200 µm.

13. Electrode selon la revendication 10, **caractérisée en ce que** ledit polymère est du parylène.

14. Electrode selon la revendication 13, **caractérisée en ce que** l'épaisseur du parylène présente une valeur comprise entre 0,5µm et 10µm.

15. Electrode selon la revendication 10, **caractérisée en ce que** ledit métal est un métal noble, ou un alliage de métaux nobles.

16. Electrode selon la revendication 15, **caractérisée en ce que** la couche de métal présente une épaisseur d'au moins 400 nm.

17. Electrode selon l'une quelconque des revendications 10 à 16, **caractérisée en ce qu'**elle est recouverte, à l'exception des plots de contact (20), par une couche (8) d'un matériau protecteur.

18. Electrode selon la revendication 17, **caractérisée en ce que** ledit matériau protecteur est du parylène.

19. Electrode selon la revendication 18, **caractérisée en ce que** la couche de parylène a une épaisseur d'au moins 1 µm.

## Patentansprüche

1. Verfahren zur Herstellung einer Elektrode für medizinische Zwecke wie eine für eine Benutzung im Gehirn bestimmte Kortikalelektrode (1), in dem man für die Herstellung von besagter Elektrode einen Silikonstreifen (3) benutzt, um ein flexibles Substrat (30) zu bilden, man auf besagtes flexibles Substrat (30) eine erste Maske (5) legt, die ein Muster (6) bestimmt, das ausgelegt ist, um zumindest eine elektrische Bahn (2) mit zumindest einer Kontaktfläche (20) zu definieren, **dadurch gekennzeichnet, dass** man besagten Silikonstreifen (3) dadurch versteift, dass man auf zumindest einer seiner Seiten eine Schicht (4) eine Polymers aufträgt, und dadurch, dass besagte erste Maske (5) aus einem Blatt aus einem Metall oder aus einer Metall-Legierung hergestellt ist und man ein magnetisiertes Teil (7) auf die der Seite mit besagter Maske (5) entgegen gesetzte Seite (32) von flexiblem Substrat (30) legt, um eine Dichtheit zwischen besagtem flexiblen Substrat (30) und besagter ersten Maske (5) zu erhalten, und man eine Metallschicht auf besagtes flexibles Substrat (30) durch besagte Maske (5) hindurch mit einem physikalischen Dampfniederschlagsprozess abscheidet.

2. Verfahren nach Anspruch 1, **dadurch gekennzeichnet, dass** man einen Silikonstreifen (3) des Typs mit verstärkter Struktur verwendet.

3. Verfahren nach Anspruch 1, **dadurch gekennzeichnet, dass** man als erste Maske (5) ein Blatt aus einem in der Gruppe bestehend aus Molybdän, rostfreier Stahl, Nickel gewählten Metall oder Metall-Legierung verwendet.

4. Verfahren nach Anspruch 3, **dadurch gekennzeichnet, dass** man ein Blatt aus einem Metall oder aus einer Metall-Legierung mit einer Dicke zwischen 50µm und 200µm verwendet.

5. Verfahren nach Anspruch 1, **dadurch gekennzeichnet, dass** man, vor der Durchführung der besagten Metallablagerung, den nicht von besagter Maske (5) verdeckten Bereich des flexiblen Substrats (30) chemisch aktiviert.

6. Verfahren nach Anspruch 5, **dadurch gekennzeichnet, dass**, um besagtes flexibles Substrat (30) zu aktivieren, man dieses einem ionischen Reinigungs-Vorgang mit einer Sauerstoff- und Argon-Mischung in Plasmazustand unterzieht und man anschließend eine Schicht Titan darauf abscheidet.

7. Verfahren nach einem beliebigen der vorhergehenden Ansprüche, **dadurch gekennzeichnet, dass** man als Metall um besagte elektrische Bahn (2) zu definieren ein Edelmetall oder eine Edelmetall-Legierung verwendet.

8. Verfahren nach einem beliebigen der vorhergehenden Ansprüche, **dadurch gekennzeichnet, dass** man die aus besagtem flexiblem Substrat (30) und besagter elektrischer Bahn (2) bestehende Anordnung, mit Ausnahme der Kontaktfläche (20), mit einer Schicht (8) eines Schutzmaterials bedeckt, die durch eine zweite Maske hindurch mit einem chemischen Dampfniederschlagsprozess abgeschieden wird.

9. Verfahren nach einem beliebigen der Ansprüche 1 oder 8, **dadurch gekennzeichnet, dass** besagtes, besagte Schicht (4) oder besagtes Material von besagter Schicht (8) bildendes Polymer Parylen ist.

10. Elektrode (1) für medizinische Zwecke, erhalten durch Umsetzung des Verfahrens nach einem Beliebigen der Ansprüche 1 bis 9, wie eine für eine Benutzung im Gehirn bestimmte Kortikalelektrode (1), wobei besagte Elektrode einen Silikonstreifen (3) beträgt, der ein flexibles Substrat (30) bildet, auf dem zumindest eine Metallschicht abgeschieden wird, die ausgelegt ist, um zumindest eine elektrische Bahn (2) mit zumindest einer Kontaktfläche (20) zu definieren, **dadurch gekennzeichnet, dass** besagter Silikonstreifen (3) zumindest auf einer seiner Seiten von einer Schicht (4) eines versteifenden Polymers bedeckt ist.

11. Elektrode nach Anspruch 10, **dadurch gekennzeichnet, dass** der verwendete Silikonstreifen (3) eine verstärkte Struktur aufweist.

12. Elektrode nach Anspruch 11, **dadurch gekennzeichnet, dass** besagter Silikonstreifen (3) eine Dicke von zumindest 200 µm aufweist.

13. Elektrode nach Anspruch 10, **dadurch gekennzeichnet, dass** besagtes Polymer Parylen ist.

14. Elektrode nach Anspruch 13, **dadurch gekennzeichnet, dass** die Dicke des Parylens einen Wert zwischen 0,5 µm und 10 µm beträgt.

15. Elektrode nach Anspruch 10, **dadurch gekennzeichnet, dass** besagtes Metall ein Edelmetall oder eine Edelmetall-Legierung ist.

16. Elektrode nach Anspruch 15, **dadurch gekennzeichnet, dass** die Metallschicht eine Dicke von zumindest 400 nm aufweist.

17. Elektrode nach einem beliebigen der Ansprüche 10 bis 16, **dadurch gekennzeichnet, dass** sie mit Ausnahme der Kontaktflächen (20) von einer Schicht eines Schutzmaterials (8) bedeckt ist.

18. Elektrode nach Anspruch 17, **dadurch gekennzeichnet, dass** besagtes Schutzmaterial Parylen ist.

19. Elektrode nach Anspruch 18, **dadurch gekennzeichnet, dass** die Parylenschicht eine Dicke von zumindest 1 µm aufweist.

## Claims

1. Process for manufacturing an electrode for medical use, such as a cortical electrode (1) intended for use at brain level, in which, to manufacture said electrode, one uses a silicone strip (3) to form a flexible substrate (30), one places on said flexible substrate (30) a first mask (5) determining a pattern (6) arranged to define at least one electrical track (2) having at least one contact pad (20), **characterized in that** one stiffens said silicone strip (3) by depositing on at least one of its sides a layer (4) of a polymer, and **in that** said first mask (5) is made from a sheet of a metal or of an alloy of metals and one arranges a magnetized part (7) on the side (32) of the flexible substrate (30) opposite to the side on which said mask (5) is applied, in order to achieve tightness between said flexible substrate (30) and said first mask (5), and one deposits a metal layer on said flexible substrate (30) through said first mask (5) by means of a physical vapor deposition technique.

2. Process according to claim 1, **characterized in that** one uses a silicone strip (3) of the type having a reinforced structure.

3. Process according to claim 1, **characterized in that** one uses for the first mask (5) a sheet of a metal or of an alloy of metals chosen in the group including molybdenum, stainless steel, nickel.

4. Process according to claim 3, **characterized in that** one uses a sheet of a metal or of an alloy of metals having a thickness included between 50µm and 200µm.

5. Process according to claim 1, **characterized in that**, prior to the step consisting in carrying out said metal deposit, one activates chemically the area of the flexible substrate (30) that is not covered by said mask (5).

6. Process according to claim 5, **characterized in that**, in order to activate said flexible substrate (30), one subjects it to an ionic cleaning step carried out by means of a mix of oxygen and argon in the plasma state, and one then deposits a layer of titanium on it.

7. Process according to any of the previous claims, **characterized in that** the metal used to define said electrical track (2) is a noble metal or an alloy of noble metals.

8. Process according to any of the previous claims, **characterized in that** one covers the set formed by said flexible substrate (30) and said electrical track (2), except for the contact pad (20), with a layer (8) of a protective material, deposited through a second mask by means of a chemical vapor deposition technique.

9. Process according to any of claims 1 or 8, **characterized in that** said polymer forming said layer (4) or said protective material forming said layer (8) is parylene.

10. Electrode (1) for medical use, obtained by the implementation of the process according to any of claims 1 to 9, such as a cortical electrode (1) intended to be used at brain level, said electrode comprising a silicone strip (3) forming a flexible substrate (30), on which at least one metal layer arranged to define at least one electrical track (2) having at least one contact pad (20) is deposited, **characterized in that** said silicone strip (3) is covered on at least one of its sides with a layer (4) of a stiffening polymer.

11. Electrode according to claim 10, **characterized in that** said silicone strip (3) used has a reinforced structure.

12. Electrode according to claim 11, **characterized in that** said silicone strip (3) has a thickness of at least 200 µm.

13. Electrode according to claim 10, **characterized in that** said polymer is parylene.

14. Electrode according to claim 13, **characterized in that** the thickness of the parylene has a value included between 0.5µm and 10µm.

15. Electrode according to claim 10, **characterized in that** said metal is a noble metal, or an alloy of noble metals.

16. Electrode according to claim 15, **characterized in that** the metal layer has a thickness of at least 400 nm.

17. Electrode according to any of claims 10 to 16, **characterized in that** it is covered, except for the contact pads (20), with a layer (8) of a protective material.

18. Electrode according to claim 17, **characterized in that** said protective material is parylene.

19. Electrode according to claim 18, **characterized in that** the parylene layer has a thickness of at least 1 µm.
